# EUROPEAN PATENT SPECIFICATION

(11) **EP 1 477 198 B1**
(45) Date of publication and mention of the grant of the patent: **18.04.2007**
(21) Application number: 04251916.5
(22) Date of filing: 03.03.2004
(51) Int. Cl.: A61M 16/04

(54) **Tracheal tube**
Trachealtubus
Tube trachéal

(30) Priority: 04.03.2003 GB 0304851
(43) Date of publication of application: 17.11.2004
(73) Proprietor: Intersurgical AG, Vaduz (LI)
(72) Inventor: Jassell, Surinderjit Kumar, Northolt, Middlesex UB5 6DE (GB)
(74) Representative: Jones, Stephen Anthony

(56) References cited:
- EP-A- 1 043 039
- WO-A-03/101516
- US-A- 5 791 341
- US-A- 6 079 409
- US-A- 6 135 111
- US-A1- 2001 017 135

## Description

This invention relates to respiratory aids and, in particular, to oropharyngeal airways of the type known as Guedel airways.

Respiratory aids include tubular devices that are inserted into a patient's body so as to provide an airway through which the patient can breathe. One such device is a so-called Guedel or oropharyngeal airway which includes a curved tube that is inserted into a patient's mouth and extends, once inserted, into the oropharynx. A Guedel airway prevents the tongue and epiglottis from falling backwards, and also prevents the pharynx collapsing following anaesthesia, thereby maintaining the patency of the upper airway of an unintubated patient.

Guedel airways are designed in different sizes to fit patients from neonatals to adults. They consist of a curved air channel that is flattened anteroposteriorly and curved laterally (arched). A protective flange at the oral end is provided to ensure that the device is secure in use, and does not slip into the oral cavity. The portion that fits between the teeth is typically made of hardened plastic to ensure that the air channel is not occluded if the patient bites down on the airway. An additional hardened plastic insert is typically used to add strength, and this insert is often colour-coded to denote the size of the airway.

Problems that may be encountered with Guedel airways include trauma to the oral tissues during insertion of the device, trauma to the teeth or dental crowns of the anaesthetised patient as the patient bites down on the device, and gagging or vomiting due to inappropriate choice of airway material and/or size. Also, the additional colour-coded plastics inserts may become detached from the airway.

A tracheostomy tube having the features defined in the preamble of Claim 1 is disclosed in US-A-6135111.

There has now been devised an improved respiratory aid which overcomes or substantially mitigates the above-mentioned and/or other disadvantages associated with the prior art.

According to the invention, there is provided a Guedel airway comprising a tubular airway having a first end adapted, in use, to be retained between a patient's teeth and a second end extending, in use, into the patient's oropharynx, the first end of the tubular airway being formed with a flange that assists with placement and retention of the tubular airway during use, characterised in that a portion of the first end of the tubular airway is coated with, and by injection moulding bonded to, an elastomeric material.

The respiratory aid according to the invention is advantageous principally in that it reduces the likelihood of injury to the patient during use. In particular, the elastomeric coating reduces the likelihood of trauma to the patient's teeth and dental crowns when the patient bites down on the respiratory aid.

The coating of elastomeric material at the first end of the airway preferably extends a sufficient distance along the airway that it covers the part of the airway that, in normal use, might be gripped between a patient's teeth.

The second end of the airway is preferably formed in, or coated with, elastomeric material. Most preferably, the leading edge of the second end of the airway is formed in, or coated with, elastomeric material. The leading edge of the airway, as the airway is inserted into the patient's oropharynx, is therefore less likely to cause damage or trauma to the patient's oral tissues.

The respiratory aid according to the invention has the general form of an oropharyngeal airway of the type known as a Guedel airway.

The flange will generally extend from the area of the first end of the airway that is coated with elastomeric material and is itself preferably coated with the elastomeric material, at least on the face of the flange that faces, in use, the patient's teeth.

The airway part of the respiratory aid is preferably formed in a rigid plastics material. By "rigid plastics material" is meant a plastics material that retains its shape and form during normal use of the respiratory aid without significant deformation. The plastics material is preferably sufficiently strong to prevent collapse of the tubular airway during use but is also preferably flexible to a certain extent so as to facilitate insertion of the respiratory aid and to reduce the likelihood of trauma to the oral tissues when the patient moves and the shape of the upper airway changes as a result. The rigid plastics material is preferably a polyolefin, such as polypropylene or polyethylene, and is most preferably polypropylene.

The elastomeric material is preferably a thermoplastic elastomeric material that is suitable for medical use. Additionally, the elastomeric material may include a lubricating additive, such as a wax additive, that reduces the coefficient of friction of the surface of the elastomeric material. The lubricating additive reduces the likelihood of oral trauma by reducing the friction between the elastomeric material and the internal surface of the upper airway.

The respiratory aid preferably comprises a single, unitary component. The respiratory aid is preferably formed using a two-shot injection moulding process, which involves injection moulding the rigid plastics material and subsequently injection moulding the elastomeric material onto the rigid plastics material. This two-shot injection moulding process bonds the elastomeric material to the external surface of the rigid plastics material. The respiratory aid is most preferably formed with a number of regions of elastomeric material, eg the elastomeric coating at the first end of the airway and elastomeric material applied to the second end, and these regions are most preferably formed simultaneously. Thus, the elastomeric coating at the first end of the airway and the elastomeric material applied to the second end may be joined by a web or line of elastomeric material extending along the length of the airway, most preferably along the concave surface of the curved airway.

As for a conventional Guedel airway, the respiratory aid preferably has dimensions that comply with the International Standard for oropharyngeal airways. The length of the respiratory aid is dependent upon the size of patient but the respiratory aid generally has a length of between 30 and 120mm. The tubular airway is preferably curved along its length, typically through an angle of between 90° and 130°, for example 120°.

The tubular airway preferably has an elliptical cross-section, which gives the tubular airway greater strength compared with the tubular airways of conventional Guedel airways which typically have flatter cross-sections. The ratio of the internal depth of the tubular airway to its internal width is preferably 0.3 or greater, eg in the range 0.3 to 0.7. Most preferably, the ratio of the internal depth of the tubular airway to its internal width is greater at the first end of the tubular airway than at the second end of the tubular airway. The difference between these two ratios is typically in the range 0.1 to 0.3, for example approximately 0.15.

The flange preferably extends outwardly from the periphery of the first end of the airway. Most preferably, the flange extends perpendicularly to the first end of the airway.

The surface of the flange, most preferably the surface that, in use, is directed outwardly from the patient's mouth, preferably includes an indication of the size of the respiratory aid. Most preferably, this indication is a symbol, eg a number, moulded in the plastics material of the flange. Most preferably, the symbol is formed in elastomeric material in the same stage of a two-shot injection moulding process as that in which the elastomeric coating is applied to the first end of the airway. In addition to the size-indication symbol, the elastomeric material may also contain a pigment, differently pigmented elastomeric materials being used to indicate respiratory aids of different standard sizes. Thus, the elastomeric material may be used to colour-code the size of the respiratory aid.

The invention will now be described in greater detail, by way of illustration only, with reference to the accompanying drawings, in which
Figure 1 is a rear perspective view of a Guedel airway according to the present invention;
Figure 2 is a side view of the Guedel airway;
Figure 3 is a front perspective view of the Guedel airway; and
Figure 4 is a plan view of the Guedel airway.

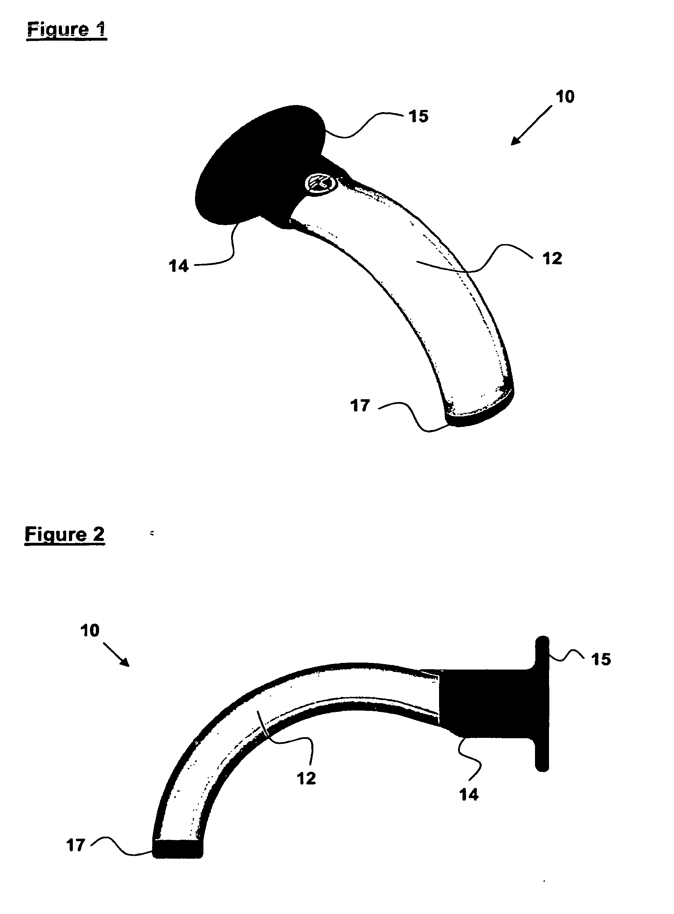

Figures 1 to 4 show a Guedel airway according to the present invention, which is generally designated 10. The Guedel airway 10 comprises a tubular airway 12 with a mouth portion 14.

The airway 12 is curved along its length through an angle somewhat greater than 90°, specifically approximately 120°, and has an elliptical cross-section. The ratio of the internal depth of the airway 12 to its internal width is approximately 0.38 at the end of the airway remote from the mouth portion 14 and approximately 0.53 at the mouth portion 14. The airway 12 is curved in the plane of the minor axis of the elliptical cross-section, along the length of the tubular airway 12. The airway 12 has a wall thickness of approximately 2mm.

At a first end of the airway 12, the airway is formed with a terminal, peripheral flange 15. The mouth portion 14 of the airway 12 adjacent to the flange 15 is orientated perpendicularly to the other end of the airway 12 and has walls of relatively greater thickness than the rest of the airway 12. The mouth portion 14 of the airway 12 carries a coating of an elastomeric material which also extends over one side of the flange 15 and the peripheral rim of the flange 15.

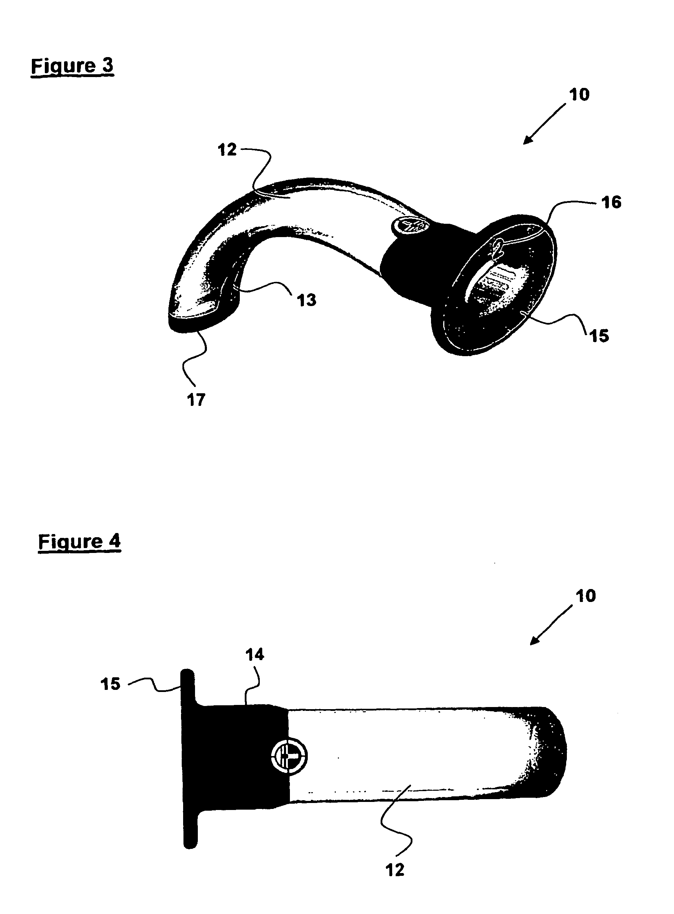

The front surface of the flange 15, as shown in Figure 3, carries a number, formed in the elastomeric material that coats the mouth portion 14 of the airway 12, which indicates the size of the Guedel airway 10. The size 2 Guedel airway 10 shown in the Figures is approximately 85mm in overall length, 50mm in overall height and 40mm in overall width.

The end of the airway 12 remote from the flange 15 is also provided with a coating of the elastomeric material 17, such that when the device is inserted into a patient's mouth the elastomeric material forms the leading edge of the Guedel airway 10.

The elastomeric coating on the mouth portion 14 of the airway 12 and the elastomeric coating 17 at the other end of the airway 12 are formed in the same moulding operation, which is the second stage of a two-shot injection moulding process. An elongate track 13 extends along the longitudinal axis of the external surface of the airway 12, on its concave side, ie the lower surface as viewed in Figure 3. This track 13 connects the elastomeric material at each end of the device and allows molten elastomeric material to flow along it during the moulding process.

The Guedel airway 10 is manufactured by injection moulding. The tubular airway 12 and mouth portion 14 are injection moulded as a single component in a suitably strong and relatively rigid plastics material, such as polypropylene. Once the tubular airway 12 and mouth portion 14 have been moulded, the elastomeric material is injection moulded onto the surface of the rigid plastics component. The areas of the tubular airway 12 and mouth portion 14 to which the elastomeric material is applied are recessed, such that the surface of the elastomeric material lies flush with adjacent surfaces of the tubular airway 12 and mouth portion 14. This process is a so-called double-shot injection moulding process. Since the elastomeric component is moulded directly onto the tubular airway 12 and mouth portion 14, the elastomeric component becomes bonded to the rigid plastics component. The elastomeric material is preferably a thermoplastic elastomer (TPE) which includes a wax or lubricating additive. The wax or lubricating additive helps to reduce damage to the oral tissues during insertion of the device, by reducing the friction between the elastomeric material and the inside of the mouth.

In use, the Guedel airway 10 is inserted into a patient's mouth with the end of the airway 12 remote from the mouth portion 14 entering first. The Guedel airway 10 is inserted in an inverted orientation so that, once inserted a certain distance, the tubular airway 12 curves towards the upper surface of the patient's upper airway. The Guedel airway 10 is then rotated through 180° and inserted further into the patient's mouth so that the airway 12 curves downwards into the oropharynx. The Guedel airway 10 is positioned so that the patient's teeth rest on the elastomeric coating of the mouth portion 14 and the protective flange 15 is located externally of the patient's mouth, adjacent to the patient's teeth. The flange 15 prevents the Guedel airway 10 from slipping further into the patient's airway.

The elastomeric end portion 17 reduces the likelihood of trauma to the oral tissues when inserting the Guedel airway into the patient's mouth. The elastomeric coating of the mouth portion 14 and protective flange 15 reduce the likelihood of tooth and crown breakage by allowing the elastomeric material to cushion a patient's bite. The wax or other lubricating additive further facilitates insertion of the device, and reduces the likelihood of damage or trauma to the adjacent tissue, by reducing the friction between the elastomeric material and the oral surfaces.

Further advantages of the Guedel airway according to the invention include the elliptical cross-sectional shape of the tubular airway 12 and mouth portion 14 giving the Guedel airway 10 greater strength compared to conventional Guedel airways which typically have flatter cross-sections. In addition, the mouth portion 14 has a relatively greater wall thickness compared to the rest of the tubular airway 12, which provides the strength to withstand the bite of the patient, and the size is indicated on the Guedel airway 10. A separate insert is therefore not required either to provide additional strength for the mouth portion 14 or to indicate the size of the Guedel airway 10. This is advantageous because it allows the internal dimensions of the mouth portion 14 to be much greater than when a separate insert is required, thereby reducing the resistance to air flow through the mouth portion 14. A further advantage is that a separate insert may be misplaced, thereby leading to confusion over the size of the Guedel airway as well as a reduction in the strength of the airway. In addition, differently-pigmented elastomeric materials may be used in different sizes of airway, in order to colour-code the sizes.

## Claims

1. A Guedel airway (10) comprising a tubular airway (12) having a first end (14) adapted, in use, to be retained between a patient's teeth and a second end (17) extending, in use, into the patient's oropharynx, the first end of the tubular airway being formed with a flange that assists with placement and retention of the tubular airway during use,
**characterised in that** a portion of the first end (14) of the tubular airway (12) is coated with, and by injection moulding bonded to, an elastomeric material.

2. A Guedel airway (10) as claimed in Claim 1, wherein the coating of elastomeric material at the first end (14) of the tubular airway (12) extends a sufficient distance along the tubular airway (12) that it covers the part of the tubular airway (12) that, in normal use, might be gripped between a patient's teeth.

3. A Guedel airway (10) as claimed in Claim 1 or Claim 2, wherein the second end (17) of the tubular airway (12) is formed in, or coated with, elastomeric material.

4. A Guedel airway (10) as claimed in any preceding claim, wherein the elastomeric material is a thermoplastic elastomeric material that is suitable for medical use.

5. A Guedel airway (10) as claimed in any preceding claim, wherein the elastomeric material includes a lubricating additive that reduces the coefficient of friction of the surface of the elastomeric material.

6. A Guedel airway (10) as claimed in any preceding claim, wherein the tubular airway (12) of the respiratory aid (10) is formed in a rigid plastics material.

7. A Guedel airway (10) as claimed in Claim 6, wherein the rigid plastics material is a polyolefin.

8. A Guedel airway (10) as claimed in any preceding claim, wherein the respiratory aid (10) comprises a single, unitary component.

9. A Guedel airway (10) as claimed in Claim 8, wherein the respiratory aid (10) is formed using a two-shot injection moulding process, which involves injection moulding a rigid plastics material and subsequently injection moulding the elastomeric material onto the rigid plastics material.

10. A Guedel airway (10) as claimed in any preceding claim, wherein the tubular airway (12) has an elliptical cross-section.

11. A Guedel airway (10) as claimed in Claim 10, wherein the ratio of the internal depth of the tubular airway (12) to its internal width is 0.3 or greater.

12. A Guedel airway (10) as claimed in Claim 10 or Claim 11, wherein the ratio of the internal depth of the tubular airway (12) to its internal width is greater at the first end (14) of the tubular airway (12) than at the second end (17) of the tubular airway (12).

13. A Guedel airway (10) as claimed in Claim 12, wherein the difference between the ratios at the first and second ends (14,17) of the tubular airway (12) is in the range 0.1 to 0.3.

14. A Guedel airway (10) as claimed in any preceding claim, wherein the flange (15) extends from the portion of the first end (14) of the tubular airway (12) that is coated with elastomeric material and is itself coated with the elastomeric material, at least on the face of the flange (15) that faces, in use, the patient's teeth.

## Patentansprüche

1. Guedel-Tubus (10), umfassend einen röhrenförmigen Luftkanal (12) mit einem ersten Ende (14), das so angepasst ist, dass es im Gebrauch zwischen den Zähnen eines Patienten gehalten werden kann, und einem zweiten Ende (17), das im Gebrauch in den Oropharynx des Patienten hineinragt, wobei das erste Ende des röhrenförmigen Luftkanals als Flansch ausgebildet ist, der im Gebrauch die Platzierung und das Halten des röhrenförmigen Luftkanals unterstützt,
**dadurch gekennzeichnet, dass** ein Teil des ersten Endes (14) des röhrenförmigen Luftkanals (12) mit einem elastischen Polymerisationsprodukt überzogen und durch Spritzgießen mit diesem verbunden ist.

2. Guedel-Tubus (10) nach Anspruch 1, wobei sich der Überzug aus elastischem Polymerisationsprodukt am ersten Ende (14) des röhrenförmigen Luftkanals (12) über ein ausreichend langes Stück des röhrenförmigen Luftkanals (12) erstreckt, dass es den Teil des röhrenförmigen Luftkanals (12) bedeckt, der im normalen Gebrauch zwischen den Zähnen eines Patienten gehalten werden kann.

3. Guedel-Tubus (10) nach Anspruch 1 oder 2, wobei das zweite Ende (17) des röhrenförmigen Luftkanals (12) aus einem elastischen Polymerisationsprodukt geformt oder damit überzogen ist.

4. Guedel-Tubus (10) nach einem der vorhergehenden Ansprüche, wobei das elastische Polymerisationsprodukt ein thermoplastisches, elastisches Polymerisationsprodukt ist, das für medizinische Anwendungen geeignet ist.

5. Guedel-Tubus (10) nach einem der vorhergehenden Ansprüche, wobei das elastische Polymerisationsprodukt ein Schmiermitteladditiv enthält, das den Reibungskoeffizienten der Oberfläche des elastischen Polymerisationsproduktes herabsetzt.

6. Guedel-Tubus (10) nach einem der vorhergehenden Ansprüche, wobei der röhrenförmige Luftkanal (12) der Beatmungshilfe (10) in einem harten Kunststoff ausgebildet ist.

7. Guedel-Tubus (10) nach Anspruch 6, wobei der harte Kunststoff ein Polyolefin ist.

8. Guedel-Tubus (10) nach einem der vorhergehenden Ansprüche, wobei die Beatmungshilfe (10) aus einem einzigen, einheitlichen Bauteil besteht.

9. Guedel-Tubus (10) nach Anspruch 8, wobei die Beatmungshilfe (10) in einem zweistufigen Spritzgussverfahren hergestellt wird, welches das Spritzen eines harten Kunststoffs und das anschließende Spritzen des elastischen Polymerisationsproduktes auf den harten Kunststoff umfasst.

10. Guedel-Tubus (10) nach einem der vorhergehenden Ansprüche, wobei der röhrenförmige Luftkanal (12) einen elliptischen Querschnitt aufweist.

11. Guedel-Tubus (10) nach Anspruch 10, wobei das Verhältnis der inneren Tiefe des röhrenförmigen Luftkanals (12) zu seinem Innendurchmesser gleich 0,3 oder größer ist.

12. Guedel-Tubus (10) nach Anspruch 10 oder 11, wobei das Verhältnis der inneren Tiefe des röhrenförmigen Luftkanals (12) zu seinem Innendurchmesser am ersten Ende (14) des röhrenförmigen Luftkanals (12) größer als am zweiten Ende (17) des röhrenförmigen Luftkanals (12) ist.

13. Guedel-Tubus (10) nach Anspruch 12, wobei die Differenz zwischen den Verhältnissen an den ersten und zweiten Enden (14, 17) des röhrenförmigen Luftkanals (12) im Bereich von 0,1 bis 0,3 liegt.

14. Guedel-Tubus (10) nach einem der vorhergehenden Ansprüche, wobei der Flansch (15) von dem Teil des ersten Endes (14) des röhrenförmigen Luftkanals (12) wegragt, der mit elastischem Polymerisationsprodukt überzogen ist und wobei der Flansch selbst mit elastischem Polymerisationsprodukt überzogen ist, zumindest an der Seite des Flansches (15), die im Gebrauch zu den Zähnen des Patienten weist.

## Revendications

1. Tube de Guedel (10) comprenant une sonde tubulaire (12) ayant une première extrémité (14) adaptée à l'usage pour être retenue entre les dents d'un patient et une seconde extrémité (17) s'étendant à l'usage dans l'oropharynx du patient, la première extrémité de la sonde tubulaire étant formée avec un rebord qui aide à la mise en place et à la retenue de la sonde tubulaire pendant l'usage,
**caractérisé en ce qu'**une partie de la première extrémité (14) de la sonde tubulaire (12) est recouverte avec, et reliée par moulage à injection à un matériau élastomère.

2. Tube de Guedel (10) selon la revendication 1, dans lequel le revêtement du matériau élastomère au niveau de la première extrémité (14) de la sonde tubulaire (12) s'étend sur une distance suffisante le long de la sonde tubulaire (12) qui recouvre la partie de la sonde tubulaire (12) qui, lors d'une utilisation normale, peut être saisie entre les dents d'un patient.

3. Tube de Guedel (10) selon la revendication 1 ou la revendication 2, dans lequel la seconde extrémité (17) de la sonde tubulaire (12) est formée dans, ou recouverte avec un matériau élastomère.

4. Tube de Guedel (10) selon l'une quelconque des revendications précédentes, dans lequel le matériau élastomère est un matériau élastomère thermoplastique qui est approprié pour l'utilisation médicale.

5. Tube de Guedel (10) selon l'une quelconque des revendications précédentes, dans lequel le matériau élastomère comprend un additif lubrifiant qui réduit le coefficient de frottement de la surface du matériau élastomère.

6. Tube de Guedel (10) selon l'une quelconque des revendications précédentes, dans lequel la sonde tubulaire (12) de l'aide respiratoire (10) est formée avec une matière plastique rigide.

7. Tube de Guedel (10) selon la revendication 6, dans lequel la matière plastique rigide est une polyoléfine.

8. Tube de Guedel (10) selon l'une quelconque des revendications précédentes, dans lequel l'aide respiratoire (10) comprend un seul composant unitaire.

9. Tube de Guedel (10) selon la revendication 8, dans lequel l'aide respiratoire (10) est formée en utilisant un procédé de moulage à deux coups, qui implique le moulage par injection d'une matière plastique rigide et ensuite le moulage par injection de la matière élastomère sur la matière plastique rigide.

10. Tube de Guedel (10) selon l'une quelconque des revendications précédentes, dans lequel la sonde tubulaire (12) a une section transversale elliptique.

11. Tube de Guedel (10) selon la revendication 10, dans lequel le rapport de la profondeur interne de la sonde tubulaire (12) sur sa largeur interne est de 0,3 ou plus.

12. Tube de Guedel (10) selon la revendication 10 ou la revendication 11, dans lequel le rapport de la profondeur interne de la sonde tubulaire (12) sur sa largeur interne est supérieur au niveau de la première extrémité (14) de la sonde tubulaire (12) qu'au niveau de la seconde extrémité (17) de la sonde tubulaire (12).

13. Tube de Guedel (10) selon la revendication 12, dans lequel la différence entre les rapports au niveau des première et seconde extrémités (14, 17) de la sonde tubulaire (12) est de l'ordre de 0,1 à 0,3.

14. Tube de Guedel (10) selon l'une quelconque des revendications précédentes, dans lequel le rebord (15) s'étend à partir de la partie de la première extrémité (14) de la sonde tubulaire (12) qui est recouverte avec le matériau élastomère et est elle-même recouverte avec le matériau élastomère, au moins sur la face du rebord (15) qui lui fait face, à l'usage, les dents du patient.
